# EUROPEAN PATENT SPECIFICATION

(11) **EP 2 329 274 B1**
(45) Date of publication and mention of the grant of the patent: **02.12.2015**
(21) Application number: 09781358.8
(22) Date of filing: 31.07.2009
(51) Int. Cl.: G01N 33/68

(54) **GLUTAMINYL CYCLASE AS A DIAGNOSTIC / PROGNOSTIC INDICATOR FOR NEURODEGENERATIVE DISEASES**
GLUTAMINYLCYCLASE ALS DIAGNOSTISCHER/PROGNOSTISCHER INDIKATOR FÜR NEURODEGENERATIVE KRANKHEITEN
GLUTAMINYL CYCLASE EN TANT QU'INDICATEUR DE DIAGNOSTIC/PRONOSTIC DE MALADIES NEUROGÉNÉRATIVES

(30) Priority: 31.07.2008 US 85154 P
(43) Date of publication of application: 08.06.2011
(73) Proprietor: Probiodrug AG, 06120 Halle/Saale (DE)
(72) Inventor: DEMUTH, Hans-Ulrich, 06120 Halle / Saale (DE); SCHILLING, Stephan, 06130 Halle / Saale (DE); KLEINSCHMIDT, Martin, 06114 Halle / Saale (DE); RAHFELD, Jens-Ulrich, 06120 Lieskau (DE); KEHLEN, Astrid, 06124 Halle/Saale (DE); HAEGELE, Monique, 04155 Leipzig (DE)
(74) Representative: Maikowski & Ninnemann
(86) International application number: PCT/EP2009/059951
(87) International publication number: WO 2010/012828

(56) References cited:
- WO-A2-2008/034891
- US-A1- 2007 191 366
- SCHILLING STEPHAN ET AL: "Glutaminyl cyclase inhibition attenuates pyroglutamate A beta and Alzheimer's disease-like pathology" NATURE MEDICINE, vol. 14, no. 10, October 2008 (2008-10), pages 1106-1111, XP002559603 ISSN: 1078-8956
- GONTSAROVA ANASTASSIA ET AL: "Glutaminyl cyclase activity is a characteristic feature of human cerebrospinal fluid" CLINICA CHIMICA ACTA, vol. 389, no. 1-2, March 2008 (2008-03), pages 152-159, XP002559604 ISSN: 0009-8981
- CYNIS HOLGER ET AL: "Amyloidogenic processing of amyloid precursor protein: Evidence of a pivotal role of glutaminyl cyclase in generation of pyroglutamate-modified amyloid-beta" BIOCHEMISTRY, vol. 47, no. 28, July 2008 (2008-07), pages 7405-7413, XP002568788 ISSN: 0006-2960
- CYNIS H ET AL: "Inhibition of glutaminyl cyclase alters pyroglutamate formation in mammalian cells" BIOCHIMICA ET BIOPHYSICA ACTA (BBA) - PROTEINS & PROTEOMICS, ELSEVIER, vol. 1764, no. 10, 1 October 2006 (2006-10-01), pages 1618-1625, XP025123183 ISSN: 1570-9639 [retrieved on 2006-10-01]
- GÜNTERT A ET AL: "High sensitivity analysis of amyloid-beta peptide composition in amyloid deposits from human and PS2APP mouse brain." NEUROSCIENCE 1 DEC 2006, vol. 143, no. 2, 1 December 2006 (2006-12-01), pages 461-475, XP002568789 ISSN: 0306-4522
- KIM TAE-SUK ET AL: "Changes in the levels of plasma soluble fractalkine in patients with mild cognitive impairment and Alzheimer's disease", NEUROSCIENCE LETTERS, vol. 436, no. 2, May 2008 (2008-05), pages 196-200, ISSN: 0304-3940
- GALIMBERTI D ET AL: "Serum MCP-1 levels are increased in mild cognitive impairment and mild Alzheimer's disease", NEUROBIOLOGY OF AGING 200612 US, vol. 27, no. 12, December 2006 (2006-12), pages 1763-1768, ISSN: 0197-4580
- DATABASE BIOSIS [Online] BIOSCIENCES INFORMATION SERVICE, PHILADELPHIA, PA, US PREV200300315150, 2002 HALKS-MILLER M ET AL: 'THE CHEMOKINE RECEPTOR CCR1 IS EXPRESSED IN THE NEURONS OF ALZHEIMERS PATIENTS AND IS AN EARLY MARKER OF DISEASE.' Database accession no. PREV200300315150 & SOCIETY FOR NEUROSCIENCE ABSTRACT VIEWER AND ITINERARY PLANNER, vol. 2002, 2002, page Abstract No. 590.1, 32ND ANNUAL MEETING OF THE SOCIETY FOR NEUROSCIENCE; ORLANDO, FLORIDA, USA; NOVEMBER 02-07, 2002

### SEQUENCE LISTING

<110> Probiodrug AG
<120> Glutaminyl cyclase as a diagnostic/prognostic indicator for neurodegenerative diseases
<130> PBD 00072/MO
<150> US 61/085,154
   <151> 2008-07-31
<160> 13
<170> PatentIn version 3.5
<210> 1
   <211> 361
   <212> PRT
   <213> Homo sapiens
<400> 1
   Met Ala Gly Gly Arg His Arg Arg Val Val Gly Thr Leu His Leu Leu
   1               5                   10                  15
   Leu Leu Val Ala Ala Leu Pro Trp Ala Ser Arg Gly Val Ser Pro Ser
               20                  25                  30
   Ala Ser Ala Trp Pro Glu Glu Lys Asn Tyr His Gln Pro Ala Ile Leu
           35                  40                  45
   Asn Ser Ser Ala Leu Arg Gln Ile Ala Glu Gly Thr Ser Ile Ser Glu
       50                  55                  60
   Met Trp Gln Asn Asp Leu Gln Pro Leu Leu Ile Glu Arg Tyr Pro Gly
   65                  70                  75                  80
   Ser Pro Gly Ser Tyr Ala Ala Arg Gln His Ile Met Gln Arg Ile Gln
                   85                  90                  95
   Arg Leu Gln Ala Asp Trp Val Leu Glu Ile Asp Thr Phe Leu Ser Gln
               100                 105                 110
   Thr Pro Tyr Gly Tyr Arg Ser Phe Ser Asn Ile Ile Ser Thr Leu Asn
   115 120 125
   Pro Thr Ala Lys Arg His Leu Val Leu Ala Cys His Tyr Asp Ser Lys
   130 135 140
   Tyr Phe Ser His Trp Asn Asn Arg Val Phe Val Gly Ala Thr Asp Ser
   145 150 155 160
   Ala Val Pro Cys Ala Met Met Leu Glu Leu Ala Arg Ala Leu Asp Lys
   165 170 175
   Lys Leu Leu Ser Leu Lys Thr Val Ser Asp Ser Lys Pro Asp Leu Ser
   180 185 190
   Leu Gln Leu Ile Phe Phe Asp Gly Glu Glu Ala Phe Leu His Trp Ser
   195 200 205
   Pro Gln Asp Ser Leu Tyr Gly Ser Arg His Leu Ala Ala Lys Met Ala
   210 215 220
   Ser Thr Pro His Pro Pro Gly Ala Arg Gly Thr Ser Gln Leu His Gly
   225 230 235 240
   Met Asp Leu Leu Val Leu Leu Asp Leu Ile Gly Ala Pro Asn Pro Thr
   245 250 255
   Phe Pro Asn Phe Phe Pro Asn Ser Ala Arg Trp Phe Glu Arg Leu Gln
   260 265 270
   Ala Ile Glu His Glu Leu His Glu Leu Gly Leu Leu Lys Asp His Ser
   275 280 285
   Leu Glu Gly Arg Tyr Phe Gln Asn Tyr Ser Tyr Gly Gly Val Ile Gln
   290 295 300
   Asp Asp His Ile Pro Phe Leu Arg Arg Gly Val Pro Val Leu His Leu
   305 310 315 320
   Ile Pro Ser Pro Phe Pro Glu Val Trp His Thr Met Asp Asp Asn Glu
                   325                 330                 335
   Glu Asn Leu Asp Glu Ser Thr Ile Asp Asn Leu Asn Lys Ile Leu Gln
               340                 345                 350
   Val Phe Val Leu Glu Tyr Leu His Leu
           355                 360
<210> 2
   <211> 382
   <212> PRT
   <213> Homo sapiens
<400> 2
   Met Arg Ser Gly Gly Arg Gly Arg Pro Arg Leu Arg Leu Gly Glu Arg
   1               5                   10                  15
   Gly Leu Met Glu Pro Leu Leu Pro Pro Lys Arg Arg Leu Leu Pro Arg
               20                  25                  30
   Val Arg Leu Leu Pro Leu Leu Leu Ala Leu Ala Val Gly Ser Ala Phe
           35                  40                  45
   Tyr Thr Ile Trp Ser Gly Trp His Arg Arg Thr Glu Glu Leu Pro Leu
       50                  55                  60
   Gly Arg Glu Leu Arg Val Pro Leu Ile Gly Ser Leu Pro Glu Ala Arg
   65                  70                  75                  80
   Leu Arg Arg Val Val Gly Gln Leu Asp Pro Gln Arg Leu Trp Ser Thr
                   85                  90                  95
   Tyr Leu Arg Pro Leu Leu Val Val Arg Thr Pro Gly Ser Pro Gly Asn
               100                 105                 110
   Leu Gln Val Arg Lys Phe Leu Glu Ala Thr Leu Arg Ser Leu Thr Ala
           115                 120                 125
   Gly Trp His Val Glu Leu Asp Pro Phe Thr Ala Ser Thr Pro Leu Gly
       130                 135                 140
   Pro Val Asp Phe Gly Asn Val Val Ala Thr Leu Asp Pro Arg Ala Ala
   145                 150                 155                 160
   Arg His Leu Thr Leu Ala Cys His Tyr Asp Ser Lys Leu Phe Pro Pro
                   165                 170                 175
   Gly Ser Thr Pro Phe Val Gly Ala Thr Asp Ser Ala Val Pro Cys Ala
               180                 185                 190
   Leu Leu Leu Glu Leu Ala Gln Ala Leu Asp Leu Glu Leu Ser Arg Ala
           195                 200                 205
   Lys Lys Gln Ala Ala Pro Val Thr Leu Gln Leu Leu Phe Leu Asp Gly
       210                 215                 220
   Glu Glu Ala Leu Lys Glu Trp Gly Pro Lys Asp Ser Leu Tyr Gly Ser
   225                 230                 235                 240
   Arg His Leu Ala Gln Leu Met Glu Ser Ile Pro His Ser Pro Gly Pro
                   245                 250                 255
   Thr Arg Ile Gln Ala Ile Glu Leu Phe Met Leu Leu Asp Leu Leu Gly
               260                 265                 270
   Ala Pro Asn Pro Thr Phe Tyr Ser His Phe Pro Arg Thr Val Arg Trp
           275                 280                 285
   Phe His Arg Leu Arg Ser Ile Glu Lys Arg Leu His Arg Leu Asn Leu
       290                 295                 300
   Leu Gln Ser His Pro Gln Glu Val Met Tyr Phe Gln Pro Gly Glu Pro
   305                 310                 315                 320
   Phe Gly Ser Val Glu Asp Asp His Ile Pro Phe Leu Arg Arg Gly Val
                   325                 330                 335
   Pro Val Leu His Leu Ile Ser Thr Pro Phe Pro Ala Val Trp His Thr
               340                 345                 350
   Pro Ala Asp Thr Glu Val Asn Leu His Pro Pro Thr Val His Asn Leu
           355                 360                 365
   Cys Arg Ile Leu Ala Val Phe Leu Ala Glu Tyr Leu Gly Leu
       370                 375                 380
<210> 3
   <211> 364
   <212> PRT
   <213> Homo sapiens
<400> 3
<210> 4
   <211> 481
   <212> PRT
   <213> Homo sapiens
<400> 4
<210> 5
   <211> 359
   <212> PRT
   <213> Homo sapiens
<400> 5
   Ala Ala Met Arg Ser Gly Gly Arg Gly Arg Pro Arg Leu Arg Leu Gly
   1 5 10 15
   Glu Arg Gly Leu Met Glu Pro Leu Leu Pro Pro Lys Arg Arg Leu Leu
   20 25 30
   Pro Arg Val Arg Leu Leu Pro Leu Leu Leu Ala Leu Ala Val Gly Ser
   35 40 45
   Ala Phe Tyr Thr Ile Trp Ser Gly Trp His Arg Arg Thr Glu Glu Leu
       50                  55                  60
   Pro Leu Gly Arg Glu Leu Arg Val Pro Leu Ile Gly Ser Leu Pro Glu
   65                  70                  75                  80
   Ala Arg Leu Arg Arg Val Val Gly Gln Leu Asp Pro Gln Arg Leu Trp
                   85                  90                  95
   Ser Thr Tyr Leu Arg Pro Leu Leu Val Val Arg Thr Pro Gly Ser Pro
               100                 105                 110
   Gly Asn Leu Gln Val Arg Lys Ala Ala Pro Val Thr Leu Gln Leu Leu
           115                 120                 125
   Phe Leu Asp Gly Glu Glu Ala Leu Lys Glu Trp Gly Pro Lys Asp Ser
       130                 135                 140
   Leu Tyr Gly Ser Arg His Leu Ala Gln Leu Met Glu Ser Ile Pro His
   145                 150                 155                 160
   Ser Pro Gly Pro Thr Arg Ile Gln Ala Ile Glu Leu Phe Met Leu Leu
                   165                 170                 175
   Asp Leu Leu Gly Ala Pro Asn Pro Thr Phe Tyr Ser His Phe Pro Arg
               180                 185                 190
   Thr Val Arg Trp Phe His Arg Leu Arg Ser Ile Glu Lys Arg Leu His
           195                 200                 205
   Arg Leu Asn Leu Leu Gln Ser His Pro Gln Glu Val Met Tyr Phe Gln
       210                 215                 220
   Pro Gly Glu Pro Phe Gly Ser Val Glu Asp Asp His Ile Pro Phe Leu
   225                 230                 235                 240
   Arg Arg Gly Val Pro Val Leu His Leu Ile Ser Thr Pro Phe Pro Ala
                   245                 250                 255
   Val Trp His Thr Pro Ala Asp Thr Glu Val Asn Leu His Pro Pro Thr
               260                 265                 270
   Val His Asn Leu Cys Arg Ile Leu Ala Val Phe Leu Ala Glu Tyr Leu
           275                 280                 285
   Gly Leu Arg Ala Trp Pro Met Thr Val Glu Arg Thr Val Arg Glu Lys
       290                 295                 300
   Val Pro Ala Gly Ala Ser Glu Ala Gln Ala Gly Ser Ala Gly Val Leu
   305                 310                 315                 320
   Val Cys Pro Phe His Thr Phe Val Ser Leu Cys Tyr Asn Trp Lys Thr
                   325                 330                 335
   Phe Phe Leu Leu Ile Val Ser Ser Cys His Pro Ser Arg Thr Gly Lys
               340                 345                 350
   Arg Pro Leu Trp Asp Asp Ser
           355
<210> 6
   <211> 42
   <212> PRT
   <213> Homo sapiens
<400> 6
<210> 7
   <211> 40
   <212> PRT
   <213> Homo sapiens
<400> 7
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 8
   <211> 40
   <212> PRT
   <213> Homo sapiens
<400> 8
   Glu Phe Arg His Asp Ser Gly Tyr Glu Val His His Gln Lys Leu Val
   1 5 10 15
   Phe Phe Ala Glu Asp Val Gly Ser Asn Lys Gly Ala Ile Ile Gly Leu
   20 25 30
   Met Val Gly Gly Val Val Ile Ala
   35 40
<210> 9
   <211> 38
   <212> PRT
   <213> Homo sapiens
<400> 9
   Glu Phe Arg His Asp Ser Gly Tyr Glu Val His His Gln Lys Leu Val
   1 5 10 15
   Phe Phe Ala Glu Asp Val Gly Ser Asn Lys Gly Ala Ile Ile Gly Leu
   20 25 30
   Met Val Gly Gly Val Val
   35
<210> 10
   <211> 38
   <212> PRT
   <213> Homo sapiens
<400> 10
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly
   35
<210> 11
   <211> 36
   <212> PRT
   <213> Homo sapiens
<400> 11
   Glu Phe Arg His Asp Ser Gly Tyr Glu Val His His Gln Lys Leu Val
   1 5 10 15
   Phe Phe Ala Glu Asp Val Gly Ser Asn Lys Gly Ala Ile Ile Gly Leu
   20 25 30
   Met Val Gly Gly
   35
<210> 12
   <211> 34
   <212> PRT
   <213> Homo sapiens
<400> 12
   Glu Ala Ser Asn Cys Phe Ala Ile Arg His Phe Glu Asn Lys Phe Ala
   1 5 10 15
   Val Glu Thr Leu Ile Cys Ser Arg Thr Val Lys Lys Asn Ile Ile Glu
   20 25 30
   Glu Asn
<210> 13
   <211> 34
   <212> PRT
   <213> Homo sapiens
<400> 13
   Glu Ala Ser Asn Cys Phe Ala Ile Arg His Phe Glu Asn Lys Phe Ala
   1               5                   10                  15
   Val Glu Thr Leu Ile Cys Phe Asn Leu Phe Leu Asn Ser Gln Glu Lys
               20                  25                  30
   His Tyr

## Description

### FIELD OF THE INVENTION

The present invention relates to a method for predicting, diagnosing and prognosticating a neurodegenerative disease, such as Alzheimer's disease (AD), Mild Cognitive Impairment (MCI) and neurodegeneration in Down's syndrome (NDS) using glutaminyl cyclase (QC) as a diagnostic/prognostic indicator.

### BACKGROUND OF THE INVENTION

Alzheimer Disease (AD) is a neurodegenerative disease that causes dementia. The terms "Alzheimer Disease" and "Alzheimer's Disease" are both utilized in the art, these terms being equivalent and are used interchangeably here and elsewhere. The period from first detection of AD to termination can range from a few years to 15 years, during which time the patient progressively suffers loss of both mental function and control of bodily functions. There is significant variability in the progress of the disease. While the majority of patients have a gradual, inexorable progression (losing on average 3 to 4 points on the 30 point Folstein mini-mental state score annually), approximately 30% of AD cases have a prolonged stable initial plateau phase lasting several years (Haxby J. V., et al., Individual trajectories of cognitive decline in patients with dementia of the Alzheimer type, J. Clin. Exp. Neuropsychol 14:575-592, 1992.). A subgroup of patients has a fulminant, rapidly progressive downhill course over several years (Mann, U., et al., Heterogeneity in Alzheimer's disease: Progression rate segregated by distinct neuropsychological and cerebral metabolic profiles, J. Neurol. Neurosurg. Psychiatry 55:956-959, 1992). Other patients (about 10% of cohorts) remain slowly progressive, showing only gradual decline from year to year (Grossi, D., et al . , Senile dementias, II International Symposium (pp. 97-99), Paris: John Libbey Eurotext, 1988.). The pathological, chemical and molecular bases of this heterogeneity remain undetermined. Recognition of the variability of AD progression represents an important clinical insight, and may explain the diagnostic difficulties presented by "atypical" cases. While in certain cases, there is a familial manifestation of the AD disease, it appears that the majority of AD cases are non-familial, and until recently (see below), no simple biological marker for the disease had been determined.

Current methods used to diagnose AD involve analysis of cerebrospinal fluid (CSF) or brain tissue obtained from postmortem patients. Thus, among the markers currently under consideration are those related to the proteins, which account for the features found in Alzheimer brains postmortem. The neurofibrillary tangle is composed primarily of a hyperphosphorylated tau protein, a cytoskeletal protein. The neuritic plaque contains a core of amyloid protein, much of which is a 42-amino acid peptide (Aβ₄₂) derived from proteolytic cleavage of a larger precursor protein. Another form of this protein derived from the same precursor contains only 40 amino acids (Aβ₄₀). Deposits of this protein are found in the brains of AD victims. However, alterations in tau and the aforementioned beta amyloid peptides do not occur with sufficient frequency and magnitude so as to afford diagnostic value and therefore, blood tests based on these proteins do not seem to correlate well with AD. In addition to C-terminal variability, N-terminally modified Aβ peptides are abundant (Saido, T.C. et al. Dominant and differential deposition of distinct beta-amyloid peptide species, Aβ N3(pE), in senile plaques. Neuron 14, 457-466 (1995) ; Russo, C. et al. Presenilin-1 mutations in Alzheimer's disease. Nature 405, 531-532 (2000); Saido, T.C., Yamao, H., Iwatsubo, T. & Kawashima, S. Amino- and carboxyl-terminal heterogeneity of beta-amyloid peptides deposited in human brain. Neurosci. Lett. 215, 173-176 (1996)). It appears that a major proportion of the Aβ peptides undergoes N-terminal truncation by two amino acids, exposing a glutamate residue, which is subsequently cyclized into pyroglutamate (pE), resulting in Aβ3(pE)-42 peptides (Saido, T.C. et al. Dominant and differential deposition of distinct beta-amyloid peptide species, Aβ N3(pE), in senile plaques. Neuron 14, 457-466 (1995) ; Saido, T.C., Yamao, H., Iwatsubo, T. & Kawashima, S. Amino- and carboxyl-terminal heterogeneity of beta-amyloid peptides deposited in human brain. Neurosci. Lett. 215, 173-176 (1996)). Alternatively, pE may be formed following (β'-cleavage by BACE1, resulting in Aβ N11(pE)-42 (Naslund, J. et al. Relative abundance of Alzheimer Aβ amyloid peptide variants in Alzheimer disease and normal aging. Proc. Natl. Acad. Sci. U. S. A. 91, 8378-8382 (1994); Liu, K. et al. Characterization of Aβ11-40/42 peptide deposition in Alzheimer's disease and young Down's syndrome brains: implication of N-terminally truncated Abeta species in the pathogenesis of Alzheimer's disease. Acta Neuropathol. 112, 163-174 (2006)). In particular Aβ N3(pE)-42 has been shown to be a major constituent of Aβ deposits in sporadic and familial AD (Saido, T.C. et al. Dominant and differential deposition of distinct beta-amyloid peptide species, Aβ N3(pE), in senile plaques. Neuron 14, 457-466 (1995) ; Miravalle, L. et al. Amino-terminally truncated Aβ peptide species are the main component of cotton wool plaques. Biochemistry 44, 10810-10821 (2005)).

The Aβ N3pE-42 peptides coexist with Aβ 1-40/1-42 peptides (Saido, T.C. et al. Dominant and differential deposition of distinct beta-amyloid peptide species, Abeta N3pE, in senile plaques. Neuron 14, 457-466 (1995) ; Saido, T.C., Yamao, H., Iwatsubo, T. & Kawashima, S. Amino- and carboxyl-terminal heterogeneity of beta-amyloid peptides deposited in human brain. Neurosci. Lett. 215, 173-176 (1996)), and, based on a number of observations, could play a prominent role in the pathogenesis of AD. For example, a particular neurotoxicity of Aβ N3pE-42 peptides has been outlined (Russo, C. et al. Pyroglutamate-modified amyloid beta-peptides-AbetaN3(pE)--strongly affect cultured neuron and astrocyte survival. J. Neurochem. 82, 1480-1489 (2002) and the pE-modification of N-truncated Aβ peptides confers resistance to degradation by most aminopeptidases as well as Aβ-degrading endopeptidases (Russo, C. et al. Pyroglutamate-modified amyloid beta-peptides--AbetaN3(pE)--strongly affect cultured neuron and astrocyte survival. J. Neurochem. 82, 1480-1489 (2002); Saido, T.C. Alzheimer's disease as proteolytic disorders: anabolism and catabolism of beta-amyloid. Neurobiol. Aging 19, S69-S75 (1998)). The cyclization of glutamic acid into pE leads to a loss of N-terminal charge resulting in accelerated aggregation of Aβ N3pE compared to the unmodified Aβ peptides (He, W. & Barrow, C.J. The Aβ 3-pyroglutamyl and 11-pyroglutamyl peptides found in senile plaque have greater beta-sheet forming and aggregation propensities in vitro than full-length Aβ. Biochemistry 38, 10871-10877 (1999); Schilling, S. et al. On the seeding and oligomerization of pGlu-amyloid peptides (in vitro). Biochemistry 45, 12393-12399 (2006)). Thus, reduction of Aβ N3pE-42 formation should destabilize the peptides by making them more accessible to degradation and would, in turn, prevent the formation of higher molecular weight Aβ aggregates and enhance neuronal survival.

However, for a long time it was not known how the pE-modification of Aβ peptides occurs. The present Applicant discovered that glutaminyl cyclase (QC) is capable to catalyze Aβ N3pE-42 formation under mildly acidic conditions, that specific QC inhibitors prevent Aβ N3pE-42 generation *in vitro* and that, therefore, inhibition of glutaminyl cyclase is a novel therapeutic concept for the causative treatment of Alzheimer's disease (Schilling, S., Hoffmann, T., Manhart, S., Hoffmann, M. & Demuth, H.-U. Glutaminyl cyclases unfold glutamyl cyclase activity under mild acid conditions. FEBS Lett. 563, 191-196 (2004) ; Cynis, H. et al. Inhibition of glutaminyl cyclase alters pyroglutamate formation in mammalian cells. Biochim. Biophys. Acta 1764, 1618-1625 (2006); Schilling et al. Inhibition of glutaminyl cyclase - a novel therapeutic concept for the causative treatment of Alzheimer's disease. Nature Medicine 14, 1106-1111 (2008)).

At present, there appears to be no satisfactory- diagnostic marker for existing AD, or for a subject, who although exhibiting normal cognitive responses, will inevitably, or most likely, develop AD.

Age-Associated Cognitive Decline (AACD) and Mild Cognitive Impairment (MCI) are terms used to identify individuals who experience a cognitive decline that falls short of dementia. These terms are equivalent, MCI being a more recently adopted term, and are used interchangeably throughout this application. Satisfaction of criteria (World Health Organization) for this diagnosis requires a report by the individual or family of a decline in cognitive function, which is gradual, and present at least 6 months. There may be difficulties across any cognitive domains (although memory is impaired in the vast majority of cases), and these must be supported by abnormal performance on quantitative cognitive assessments for which age and education norms are available for relatively healthy individuals (i.e., the patient is compared to normal subjects his/her own age) . Performance must be at least 1 SD below the mean value for the appropriate population on such tests. Neither dementia, nor significant depression or drug effects may be present. No cerebral or systemic disease or condition known to cause cerebral cognitive dysfunction may be present. In Applicant's experience, all patients who were classified as CDR.5 ("questionable dementia") on the Clinical Dementia rating scale and who met these exclusions, also met the criteria for AACD/MCI . About 1/3 of Alzheimer's patients have had a clearly definable period of isolated memory deficit which preceded their more global cognitive decline. (Haxby J. V., et al . , Individual trajectories of cognitive decline in patients with dementia of the Alzheimer type, J. Clin. Exp. Neuropsychology 14:575-592, 1992.) Using AACD/MCI criteria, which look at other domains in addition to memory, the percentage with an identifiable prodrome is likely higher. Fortunately, not all AACD/MCI individuals seem to decline. It appears that a significant number of these subjects show a stable, non-progressive memory deficit on testing.

Attempts at predicting the onset of AD, MCI or NDS, or monitoring their progression have met with limited success. It has been discovered by the inventors of this application that an amount of QC in a biological sample obtained from a subject that deviates from a reference amount in a control person can be positively correlated to a neurological disease state. Thus, the correlation of the presence of QC with the disease state represents a positive and more direct test for diagnosis in a patient suffering from one of the neurodegenerative diseases described above. Accordingly, the invention provides an easily administered biological sample test for predicting, diagnosing, or prognosticating AD, MCI and NDS using QC as a diagnostic marker.

### SUMMARY OF THE INVENTION

The present invention is based on the discovery that an amount of glutaminyl cyclase (QC) in a biological sample obtained from a subject suffering from AD or MCI is elevated compared to an amount of QC in the biological sample obtained from a normal (i.e. healthy) control subject. The indication that the amount of QC differs between these neurological diseases and normal controls, forms the basis for the development of a test for diagnosing AD, MCI or NDS in a subject. As such, the methods for diagnosing AD, MCI or NDS of the present invention by measuring the amount of QC in patient sample will greatly improve current clinical diagnostic assessment for patients suffering from these neurodegenerative diseases.

Based on the newly discovered differences in the amount of QC present in a biological sample obtained from a patient compared to that of a normal control, a strong correlation of the amount of QC can be made to a probable diagnosis of a neurodegenerative disease. A statistically significant elevation in the amount of QC relative to control samples is reasonably predictive that the patient has AD, NDS or MCI. A normal amount of QC as determined by an amount of QC characteristic of a control QC sample isolated from a normal age-matched population indicates that the patient does not have a neurodegenerative disease, such as AD, MCI or NDS. A positive indication of a neurodegenerative disease based on an elevated or reduced amount of QC in a biological sample relative to a normal control is generally considered together with other factors in making a definitive determination of a particular disease. Therefore, the elevated or reduced QC levels of the subject being tested will usually be considered together with other accepted clinical symptoms of AD, MCI or NDS-related conditions in making a determinative diagnosis of a neurodegenerative disease.

Thus, according to a first aspect of the invention, there is provided a method for diagnosing probable Alzheimer's Disease (AD), Neurodegeneration in Down's syndrome (NDS) or Mild Cognitive Impairment (MCI) in a subject, the method comprising:
(a) detecting the amount of glutaminyl cyclase (QC), or its isoforms, in a biological sample obtained from said subject; and
(b) comparing the detected amount of QC in the biological sample with an amount of QC characteristic of a normal control;
whereby an elevated amount of QC in said biological sample relative to the normal control is a positive indicator of AD or MCI.

According to a preferred embodiment of the invention, the biological sample is a fluid body sample such as serum, plasma, urine or cerebrospinal fluid. More preferably, the fluid body sample is plasma.

According to a further embodiment of the present invention, the amount of QC is detected either on the basis of the QC protein level or the QC mRNA level.

The amount of QC detected or quantified in a biological sample from a subject can be accomplished by any means known in the art. Such means may include, but are not limited to, for example by immunoturbidimetric assay, immunofluorescence, immunodiffusion, enzyme-linked immunosorbent assay (ELISA), radioimmunoassay (RIA), Western Blot, protein activity assay or, for the determination of the QC mRNA level, Northern Blot or polymerase chain reaction (PCR) analysis, for example real-time PCR. Also useful are high performance liquid chromatography (HPLC), mass spectrometry (MS) and gas chromatography (GC), as well as their various configurations, including gas chromatograph-mass spectrometry (GC-MS), liquid chromatography-mass spectrometry (LC-MS) and liquid-chromatography-tandem mass spectrometry (LC-MS/MS) systems.

Preferably, the amount of QC in the biological sample is detected using an antibody that binds to QC in an immunoassay format. Thus, according to a preferred embodiment of the invention, there is provided a method of diagnosing a neurodegenerative disease in a subject, the method comprising:
(a) obtaining a biological sample from said subject;
(b) contacting said biological sample with an antibody that binds to glutaminyl cyclase (QC), or its isoforms ;
(c) allowing the antibody and QC to form an immune complex; and
(d) detecting the amount of immune complex formed as an indication of the amount of QC in said biological sample; and
(e) comparing the detected amount to a normal control;
whereby a detected amount that is elevated or reduced relative to the normal control is a positive indicator of a neurodegenerative disease.

### SHORT DESCRIPTION OF THE FIGURES

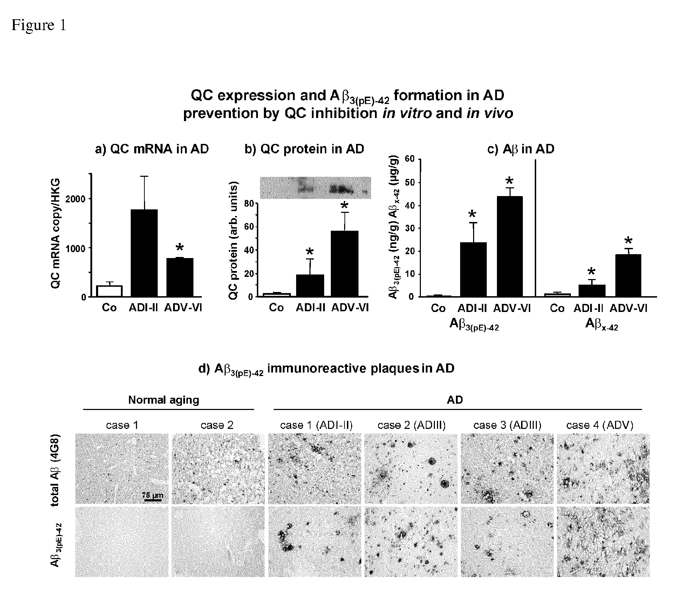

**Figure 1:** Figure 1 (a) shows the analysis of QC transcript levels applying quantitative RT-PCR. Total RNA from human neocortical brain samples (Brodmann area 22) was isolated from normally aged and AD brains of different Braak stages as indicated. The QC transcript level was normalized to house-keeping transcript concentration. Figure 1 (b) shows the Western-Blot analysis for QC from the same cases and brain region as used for QC mRNA analysis. The extraction of soluble protein was normalized to the tissue weight. Figure 1 (c) shows the quantification of Aβ N3(pE)-42 (indicated as Aβ₃(pE)-42) and of Aβ 1-42 (Aβ₁₋₄₂) concentrations from the same cases and brain region applying ELISA analysis of SDS- and formic acid extracts of human neocortical brain samples. Note the robust increase in Aβ N3(pE)-42 peptide concentrations at early AD stages compared to the much more moderate increase in Aβ 1-42 peptides. Figure 1 (d) shows the immunohistochemical detection of total Aβ peptides by the antibody 4G8 and of Aβ N3(pE)-42 peptides in Brodmann area 22 from normally aged subjects and different AD stages. Sparse Aβ plaques were detected in normal aging but these deposits lacked Aβ N3(pE)-42 immunoreactivity. At all AD stages, however, the majority of Aβ plaques contains Aβ N3(pE)-42 peptides.

### SEQUENCES OF AMYLOID PEPTIDES AND CHEMOKINES

| | |
|---|---|
| Aβ(1-42) (SEQ ID NO: 6) | |
| Aβ(1-40) (SEQ ID NO: 7) | |
| Aβ(3-42) (SEQ ID NO: 8) | |
| Aβ(3-40) (SEQ ID NO: 9) | |
| Aβ(1-38) (SEQ ID NO: 10) | |
| Aβ(3-38) (SEQ ID NO: 11) | |
| ABri (SEQ ID NO: 12) | EASNCFAIRHFENKFAVETLICSRTVKKNIIEEN |
| ADan (SEQ ID NO: 13) | EASNCFAIRHFENKFAVETLICFNLFLNSQEKHY |

### DESCRIPTION OF THE EMBODIMENTS OF THE INVENTION

The present invention provides an efficient and rapid *in vitro* method for diagnosing a neurodegenerative disease by directly detecting an amount of QC in a biological sample obtained from a subject and comparing the detected amount of QC with an amount of QC characteristic of a normal control. An elevated amount of QC in the biological sample of the subject is a positive indication of AD or MCI or NDS. Thus, as described herein, it is demonstrated that QC is consistently and significantly elevated in a biological sample of AD, NDS or MCI patients compared to normal controls. As such, the methods for diagnosing AD, MCI or NDS of the present invention by detecting or quantifying the amount of QC in a patient sample will greatly improve current clinical diagnostic assessment for patients suffering from these neurodegenerative diseases.

Accordingly, there is provided a method for assessing whether a subject may be suffering from AD, MCI or NDS using QC as a biological marker.

Glutaminyl cyclase or glutaminyl-peptide cyclotransferase (QC, EC 2.3.2.5) catalyzes the intramolecular cyclization of N-terminal glutaminyl residues into pyroglutamic acid (5-oxo-proline, pGlu*) under liberation of ammonia and the intramolecular cyclization of N-terminal glutamyl residues into pyroglutamic acid under liberation of water.

A QC was first isolated by Messer from the Latex of the tropical plant Carica papaya in 1963 (Messer, M. 1963 Nature 4874, 1299). 24 years later, a corresponding enzymatic activity was discovered in animal pituitary (Busby, W. H. J. et al. 1987 J Biol Chem 262, 8532-8536; Fischer, W. H. and Spiess, J. 1987 Proc Natl Acad Sci U S A 84, 3628-3632). For the mammalian QCs, the conversion of Gln into pGlu by QC could be shown for the precursors of TRH and GnRH (Busby, W. H. J. et al. 1987 J Biol Chem 262, 8532-8536; Fischer, W. H. and Spiess, J. 1987 Proc Natl Acad Sci U S A 84, 3628-3632). In addition, initial localization experiments of QC revealed a co-localization with its putative products of catalysis in the bovine tractus hypothalamo-hypophysalisfurther improving the suggested function in peptide hormone maturation (Bockers, T. M. et al. 1995 J Neuroendocrinol 7, 445-453). In contrast, the physiological function of the plant QC is less clear. In case of the enzyme from C. papaya, a role in the plant defence against pathogenic microorganisms was suggested (El Moussaoui, A. et al. 2001 Cell Mol Life Sci 58, 556-570). Putative QCs from other plants were identified by sequence comparisons recently (Dahl, S. W. et al. 2000 Protein Expr Purif 20, 27-36). The physiological function of these enzymes, however, is still ambiguous.

The QCs known from plants and animals show a strict specificity for L-Glutamine in the N-terminal position of the substrates and their kinetic behaviour was found to obey the Michaelis-Menten equation (Pohl, T. et al. 1991 Proc Natl Acad Sci U S A 88, 10059-10063; Consalvo, A. P. et al. 1988 Anal Biochem 175, 131-138; Gololobov, M. Y. et al. 1996 Biol Chem Hoppe Seyler 377, 395-398). A comparison of the primary structures of the QCs from C. papaya and that of the highly conserved QC from mammals, however, did not reveal any sequence homology (Dahl, S. W. et al. (2000) Protein Expr Purif 20, 27-36). Whereas the plant QCs appear to belong to a new enzyme family (Dahl, S. W. et al. (2000) Protein Expr Purif 20, 27-36), the mammalian QCs were found to have a pronounced sequence homology to bacterial aminopeptidases (Bateman, R. C. et al. 2001 Biochemistry 40, 11246-11250), leading to the conclusion that the QCs from plants and animals have different evolutionary origins.

Gostranova et al. have found that glutaminyl cyclase activity is a characteristic feature of cerebrospinal fluid in multiple sclerosis patients and controls (Gostranova et al., Clin Chim Acta. 2008 389 (1-2), pp. 152-159).

Different isoforms of QC, the glutaminyl-peptide cyclotransferase-like proteins (QPCTLs) have been observed (WO 2008/034891). These novel proteins have significant sequence similarity to glutaminyl cyclase, e.g. the QPCTL from human (further named as isoQC) (GenBank accession no. NM_017659).

Multiple isoforms of a protein, such as QC or human isoQC, can also be produced from a single gene by a variety of mechanisms, including alternative RNA splicing, post- translational proteolytic processing and cell type-specific glycosylation. Thus, the terms "glutaminyl cyclase", "QC" and "isoQC" as used herein refer to QC in its native form, as well as any of its isoforms.

The human QC or its isoforms have for example an amino acid sequence selected from the group of SEQ ID NO's: 1, 2, 3, 4 and 5.

Thus, according to a first aspect of the present invention, there is provided a method for diagnosing probable Alzheimer's Disease (AD), Neurodegeneration in Down's Syndrome (NDS) or Mild Cognitive Impairment (MCI) in a subject, the method comprising:
(a) detecting the amount of glutaminyl cyclase (QC), or an isoform thereof, in a biological sample obtained from said subject; and
(b) comparing the detected amount of QC in the biological sample with an amount of QC characteristic of a normal control;
whereby an elevated amount of QC in said biological sample relative to the normal control is a positive indicator of AD, NDS or MCI.

It has been demonstrated by inventors of the present invention that an elevated amount of QC in a biological sample may correlate with an elevated amount of N-terminally truncated and pyroglutamated amyloid beta peptides, such as for example Aβ N3pE-42 and/or Aβ N3pE-40 and/or Aβ N3pE-38.

Thus, according to a further aspect of the present invention, there is provided a method for diagnosing probable Alzheimer's Disease (AD), Neurodegeneration in Down's Syndrome (NDS) or Mild Cognitive Impairment (MCI) in a subject, the method comprising:
(a) detecting the amount of glutaminyl cyclase (QC), or an isoform thereof, in a biological sample obtained from said subject; and
(b) further detecting the amount of Aβ N3pE-X,
(c) comparing the detected amount of QC and Aβ N3pE-X in the biological sample with an amount of QC and Aβ N3pE-X characteristic of a normal control;
whereby an elevated amount of QC and Aβ N3pE-X in said biological sample relative to the normal control is a positive indicator of AD, NDS or MCI, and
wherein X is an integer selected from 38, 40 and 42.

In a preferred embodiment, X is 42.

In a further preferred embodiment, X is 40.

In a yet preferred embodiment, X is 38.

Further preferred are methods, wherein not only a single form of the N-terminally truncated and pyroglutamated amyloid beta peptides but a combination of Aβ N3pE-42 and/or Aβ N3pE-40 and/or Aβ N3pE-38 is detected together with QC.

Further preferred are methods, wherein not only a single form of the N-terminally truncated and pyroglutamated amyloid beta peptides but a combination of Aβ N3pE-42 and/or Aβ N3pE-40 and/or Aβ N3pE-38 and/or peptides occurring in familial Alzheimer's dementias, such as pGluABri or pGluADan, is detected together with QC.

"pGlu-Aβ" or "Aβ N3pE" refers to N-terminally truncated forms of Aβ, that start at the glutamic acid residue at position 3 in the amino acid sequence of Aβ, and wherein said glutamic acid residue is cyclized to form a pyroglutamic acid residue. In particular, by pGlu-Aβ as used herein are meant those fragments which are involved in or associated with the amyloid pathologies including, but not limited to, pGlu-Aβ 3-38, pGlu-Aβ 3-40, p-Glu-Aβ 3-42.

In a further preferred embodiment, any of the aforementioned methods for diagnosing Alzheimer's Disease (AD), Neurodegeneration in Down's Syndrome (NDS) or Mild Cognitive Impairment (MCI) may also be performed in vitro in a biological sample of a subject.

The term "subject" refers to a mammal which is afflicted with, or suspected to be afflicted with a neurogenerative disease such as AD, MCI or NDS. Preferably, "subject" refers to a human.

The term "biological sample" refers to any source of biological material, including, but are not limited to, peripheral blood, plasma, lymphocytes, cerebrospinal fluid, urine, saliva, epithelia, fibroblasts, or any other sample comprising QC protein.

In a preferred embodiment, the amount of QC is detected in a body fluid sample obtained from a mammal, most preferably a human. The term "body fluid" refers to all fluids that are present in the human body including but not limited to blood, lymph, urine and cerebrospinal fluid (CSF) comprising QC. The blood sample may include a plasma sample or a serum sample, or fractions derived from these samples. The sample can be treated prior to use, such as preparing plasma from blood, diluting viscous fluids, and the like. Preferably, the plasma sample is treated with an anti -coagulant, such as EDTA.

According to a preferred embodiment of the present invention, the amount of QC is detected in a blood sample taken from the subject, more preferably a plasma sample. Thus, the present invention preferably relates to a method as described above, comprising the steps of: obtaining a plasma sample from said subject; detecting the amount of QC in the plasma sample; comparing the detected amount of QC in the plasma sample with the amount of QC in a plasma sample from a normal control, whereby an elevated amount of QC relative to the normal control is a positive indication of AD, NDS or MCI. Elevated amounts of QC have been shown to correlate with and are useful in aiding the diagnosis of AD, NDS and MCI.

An "elevated amount" of QC (or an isoform thereof) means that the amount of QC detected in the samples of the subjects is greater than the mean amount of QC characteristic of a normal control person beyond the range of experimental error, as known in the art. Preferably, the amount of QC detected in the samples of the subjects is 10 % greater than said mean amount of QC characteristic of a normal control person. More preferably, the amount of QC (or an isoform thereof) detected in the samples of the subjects is 25 % greater, or, even more preferred 50 % or 75 % greater than said mean amount of QC characteristic of a normal control person. Most preferably, the amount of QC (or an isoform thereof) detected in the samples of the subjects is several times greater than said mean amount of QC characteristic of a normal control person, e.g. 2, 3, 4, 5, 6, 7, 8, 9, 10 or more times greater.

A "normal control" is a biological sample of the same type obtained from the subject, for example that is obtained from at least one normal age-matched control person or from the patient at another time. In an embodiment, the normal control is taken from the patient at an earlier time. A normal control sample from a normal age-matched population should be isolated from an adequate population sample of healthy age matched controls with no history of AD, MCI or NDS in their family. By way of example, a plasma QC level higher than the control levels of QC, as determined by an adequate control population sample size, is indicative of AD, NDS or MCI. One of skill in the art will appreciate that the sample from the subject to be diagnosed is assessed against a normal age-matched control and that a significant elevation or reduction in the amount of QC in the subject's protein sample is determined based on comparison to the controls used in the given assay.

According to a further embodiment of the present invention, the amount of QC, or an isoform thereof, is detected either on the basis of the protein level or the mRNA level of said QC or isoform thereof.

The amount of QC detected or quantified in a subject's biological sample can be accomplished by any means known in the art. Such means may include, but are not limited to, for example by immunoturbidimetric assay, immunofluorescence, immunodiffusion, enzyme-linked immunosorbent assay (ELISA), radioimmunoassay (RIA), Western Blot, protein activity assay, or, for the determination of the QC mRNA level, Northern Blot or polymerase chain reaction (PCR) analysis, for example real-time PCR. Also useful are high performance liquid chromatography (HPLC), mass spectrometry (MS) and gas chromatography (GC), as well as their various configurations, including gas chromatograph-mass spectrometry (GC-MS), liquid chromatography-mass spectrometry (LC-MS) and liquid-chromatography-tandem mass spectrometry (LC-MS/MS) systems, to name a few.

While detection of QC can be accomplished by methods known in the art for detecting peptides, the use of immmunological detection techniques using antibodies, antibody fragments, recombinant antibodies, and the like, is preferred. Therefore, such detection of QC includes, but is not limited to, the use of antibodies, which specifically bind to QC, or its isoforms, to form an immune complex, as well as reagents for detecting the formation of the immune complex. Particularly suitable detection techniques employing one or more antibodies include immunoturbidimetric assay, immunofluorescence, immunodiffusion, ELISA, RIA and the like.

Such antibodies may be polyclonal or monoclonal. Methods to produce polyclonal or monoclonal antibodies are well known in the art. For a review, see Harlow and Lane (Harlow, E. and Lane, D., Antibodies: A Laboratory Manual, Cold Spring Harbor Laboratory Press, Cold Spring Harbor, NY, 1988) and Yelton et al . (Yelton D. E. and Scharff M. D. Monoclonal Antibodies: a powerful new tool in biology and medicine. Ann. Rev. Biochem. 50:657-680, 1981). For monoclonal antibodies, see Kohler and Milstein (Kohler G. and Milstein C, Continuous cultures of fused cells secreting antibody of predefined specificity, Nature 256:495-497, 1975). The antibodies of the invention are of any isotype, e.g., IgG or IgA, and polyclonal antibodies are of a single isotype or a mixture of isotypes.

According to a preferred embodiment of the invention, the anti-QC antibody is a monoclonal antibody. Although anti-QC antibodies are widely commercially available, antibodies for use in the various immunoassays described herein, can be produced according to standard methods.

Further, the monoclonal anti-QC antibody is capable of recognizing QC in its native form, as well as any of its isoforms. Thus, any monoclonal antibody that specifically recognizes QC, including its isoforms, can be used in said method for the quantification of QC.

Preferred are monoclonal antibodies, that specifically recognize QC but show low, or more preferably, no crossreactivity with isoforms of QC. Alternatively preferred are monoclonal antibodies that specifically recognize a particular isoform of QC but show low, or more preferably, no crossreactivity with QC.

Suitable anti-QC antibodies are, for example, those which are commercially available from Abnova (Taipei City, Taiwan), e.g. a mouse polyclonal antibody (Cat. # H00025797-B01P) and a rabbit polyclonal antibody (Cat. # H00025797-D01P).

A suitable anti-QPCTL antibody is, for example, the commercially available mouse polyclonal antibody from Abnova (Taipei City, Taiwan, Cat. # H00054814-B01P).

Also fragments derived from these monoclonal antibodies such as Fab, F(ab)_{2/} ssFv (single chain variable fragment) and other antibody-like constructs that retain the variable region of the antibody, providing they have retained the original binding properties, can be used in a method of the present invention. Such fragments are commonly generated by, for instance, enzymatic digestion of the antibodies with papain, pepsin, or other proteases. It is well known to the person skilled in the art that monoclonal antibodies, or fragments thereof, can be modified for various uses. Thus, antibodies of the invention, may be recombinant, e.g., chimeric (e.g., constituted by a variable region of murine origin associated with a human constant region), humanized (a human immunoglobulin constant backbone together with hypervariable region of animal, e.g., murine, origin), and/or single chain.

An antibody specific for QC, or its isoforms, used in a method of the present invention may be labelled by an appropriate label and identified in the biological sample based upon the presence of the label. The label allows for the detection of the antibody when it is bound to QC. Examples of labels include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), luminescent labels, enzymatic labels (e.g., horseradish peroxidase, beta-galactosidase, luciferase, alkaline phosphatase), chemiluminescent , and biotinyl groups.

Methods for conjugating or labelling the antibodies discussed above may be readily accomplished by one of ordinary skill in the art (see for example Inman, "Methods In Enzymology" , Vol. 34, Affinity Techniques, Enzyme Purification: Part B, Jakoby and Wichek (eds.), Academic Press, New York, p. 30, 1974; and Wilchek and Bayer, "The Avidin-Biotin Complex in Bioanalytical Applications," Anal. Biochem. 171:1-32, 1988).

For diagnostic applications, the anti-QC antibody is either in a free state or immobilized on a solid support, such as a tube, a bead, or any other conventional support used in the field. Immobilization is achieved using direct or indirect means. "Direct means" include passive adsorption (non-covalent binding) or covalent binding between the support and the reagent. By "indirect means" is meant that an anti-reagent compound that interacts with a reagent is first attached to the solid support. Indirect means may also employ a ligand-receptor system, for example, where a molecule such as a vitamin is grafted onto the reagent and the corresponding receptor immobilized on the solid phase. This is illustrated by the biotin-streptavidin system.

Those skilled in the art will readily understand that an immune complex is formed between QC in the biological sample and the antibody, and that any unbound material is removed prior to detecting the complex. It is understood that an antibody of the invention is used for quantifying an amount of QC in the biological sample, such as, for example, blood, plasma, lymphocytes, cerebrospinal fluid, urine, saliva, epithelia and fibroblasts.

As is known in the art, the determination of such antibody binding can be performed using a great variety of immunoassay formats including, but not limited to immunoturbidimetric assay (agglutination), enzyme-linked immunosorbent assay (ELISA) and radioimmunoassay (RIA) (see, for example, "Principles and Practice of Immunoassay" (1991) Christopher P. Price and David J. Neoman (eds), Stockton Press, New York, N. Y. and Ausubel et al . (eds) (1987) in "Current Protocols in Molecular Biology" John Wiley and Sons, New York, N. Y.). Detection may be by colormetic or radioactive methods or any other conventional methods known to one skill in the art. Other standard techniques known in the art are described in "Methods in Immunodiagnosis" , 2nd Edition, Rose and Bigazzi, eds., John Wiley and Sons, New York 1980 and Campbell et al.; "Methods of Immunology", W. A. Benjamin, Inc., 1964; U.S. Patent Nos . 4,366,241; 4,376,110; 4,517,288; and 4,837,168. For a review of the general immunoassays, see also "Methods In Cell Biology", Vol. 37, Asai, ed. Academic Press, Inc. New York (1993) ; "Basic And Clinical Immunology" 7th Edition, Stites & Terr, eds. (1991) .

Such assays for detecting QC may be a direct, indirect, competitive, or noncompetitive immunoassay as described in the art (see, for example, "Principles and Practice of Immunoassay" (1991) Christopher P. Price and David J. Neoman (eds), Stockton Press, New York, N.Y.; Ausubel et al. (eds) (1987) in "Current Protocols in Molecular Biology" John Wiley and Sons, New York, N. Y.; and Oellirich, M. 1984. J. Clin. Chem. Clin. Biochem. 22: 895-904).

Noncompetitive immunoassays are assays in which the amount of QC is directly detected. In the "sandwich" assay, for example, the anti -QC antibodies can be bound directly to a solid substrate where they are immobilized. These immobilized antibodies then capture the QC present in the biological sample. The QC thus immobilized is then bound by a labeling agent, such as a second human QC antibody bearing a label.

In a competitive immunoassay, the amount of antigen present in the biological sample is determined indirectly following addition of a known amount of labeled antigen to the sample and detecting the amount of labeled antigen bound with antibodies. For example, a known amount of, in this case, labeled QC is added to the biological sample and the sample is then contacted with anti-QC antibodies. The amount of labeled QC bound to the anti-QC antibody is inversely proportional to the concentration of QC in the biological sample. This is because the greater the amount of labeled QC detected, the less the amount of QC was available in the biological sample to compete with the labeled QC.

Diagnostic kits for carrying out the assays for diagnosing AD, MCI or NDS in a subject are also provided. Thus, the present invention can be practiced using a diagnostic kit that includes at least one antibody specific for QC, and its isoforms, as described herein as well as any reagents necessary for the detection of antibody-QC binding immune complexes. Generally, the kit may include a single antibody that specifically recognizes QC, and its isoforms. On the other hand, the kit may include a primary antibody that specifically recognizes QC, and its isoforms, as well as a secondary antibody that is conjugated with a signal -producing label and is capable of binding to the primary antibody, or at a site different from the site where the primary antibody binds. The signal -producing label linked to the secondary antibody may be, but is not limited to, an enzyme, such as horseradish peroxidase or alkaline phosphatase. The kits may further comprise other reagents for carrying out the assay such as buffers, a solid support, solutions and the like. The kit may also contain instructions for carrying out the method of the invention using one or more antibodies in diagnostic assays.

### EXAMPLES OF THE INVENTION

### Example 1: Formation of AβN3pE-42 and QC expression in vivo

A widespread QC distribution has been detected in mammalian brain with considerable expression in hippocampus and cortex. In order to assess whether QC expression in AD can be correlated with generation of Aβ N3pE-42, QC mRNA and protein concentrations were analyzed in human neocortical brain samples *post mortem* (Figure 1a, b). Intriguingly, the inventors found an upregulation of QC mRNA and protein in AD brain samples, compared to normal aging. Moreover, significant concentrations of Aβ N3pE-42 were detected in samples from AD patients in contrast to non-demented individuals supporting a role of QC in generation of Aβ N3pE-42 (Figure 1c). On the other hand, ELISA analysis revealed high Aβ x-42 concentrations in normally aged control subjects and a much smaller increase at early AD stages (Figure 1c). This observation was corroborated by immunohistochemistry applying antibodies detecting total Aβ (4G8) or specifically Aβ N3pE-42 (Figure 1d). Conspicuous immunoreactivity for Aβ was detected in brain sections from all groups. In contrast, Aβ N3pE-42 staining was absent in normal aging but specific for AD brain tissue, where Aβ N3pE-42-immunoreactive plaque load was almost as high as the total of Aβ plaque density.

### Material and methods

### Human brain tissue

The definite diagnosis of AD for all cases used in this study was based on the presence of neurofibrillary tangles and neuritic plaques in the hippocampal formation and neocortical areas and met the criteria of the National Institute of Neurologic and Communicative Disorders and Stroke (NINDS) and the Alzheimer's Disease and Related Disorders Association (ADRDA). Cortical tissue (Brodmann area 22) from the same cases was used for the quantification of QC mRNA concentrations, QC protein and Aβ N3pE-42. In total, 10 control cases and 10 AD cases each of Braak staging I-II and V-VI were analyzed. The groups were matched for gender and age (control: mean 72 years ± 6.6 years; AD I-II: mean 73 years ± 3.1 years; AD V-VI: mean 77 years ± 6.6 years). The mean post morten interval (PMI) was similar among the groups and ranged from 26 to 96 hours. The duration of PMI was neither related to the detection of QC by Western blot analysis nor to quantification of Aβ by ELISA. For QC mRNA detection by qRT-PCR, only tissue samples with a PMI below 48 hours were included.

### QC mRNA quantification and QC Western blot analysis

Tissue samples were homogenized by means of the homogenizer Precellys with 1.4 mm ceramic beads (5000 rpm, 30 sec, peqlab). RNA was isolated using the NucleoSpin RNA II kit (Macherey Nagel) according to the manufacturer's instructions. Constant 100 ng of RNA were reverse transcribed to cDNA using random primers (Roche) and Superscript II (Invitrogen). Quantitative real-time PCR was performed in a Rotorgene3000 (Corbett Research) using the QuantiTect Primer Assay for QPCT (QT00013881, Qiagen) as well as the QuantiTect SYBR Green RT-PCR kit (Qiagen). Absolute amounts of QC were determined using six dilutions of the external QC standard DNA (full length QC cloned in the pcDNA3 vector) in duplicate. For verification of the PCR, product melting curves were generated and single amplicons were confirmed by agarose gel electrophoresis. Absolute amounts were determined with the Rotorgene software version 4.6 in quantitation mode. Normalization was done against the two most stably expressed housekeeping genes HPRT and GAPDH (geNorm). For Western-Blot analysis, the brain samples (50 mg) were homogenized in buffer (1 ml) containing 10 mM Tris pH 7.5, 100 mM NaCl, 5 mM EDTA and 0.5% Triton X-100 and 10% glycerol. The tissue was homogenized by several strokes in Downs-homogenizer and subjected to 3x 10s of ultrasonic shock. The resulting homogenate was cleared by centrifugation at 20000xg for 25 min. A total of 12 µg protein of each sample was separated in Tris-Glycine SDS-PAGE. QC was detected using purified rabbit polyclonal antibodies raised against recombinant human QC. For visualization, blot membranes were incubated with secondary antibody conjugated with horseradish peroxidase (Cell Signaling) in TBS-T containing 5 % (w/v) dry milk and subsequently developed using the SuperSignal West Pico System (Pierce) according to the manufacturer's protocol.

### Example 2: Determination of QC activity

### Fluorometric assays

All measurements were performed with a BioAssay Reader HTS-7000Plus for microplates (Perkin Elmer) at 30 °C. QC activity was evaluated fluorometrically using H-Gln-bNA. The samples consisted of 0.2 mM fluorogenic substrate, 0.25 U pyroglutamyl aminopeptidase (Unizyme, Hørsholm, Denmark) in 0.2 M Tris/HCl, pH 8.0 containing 20 mM EDTA and an appropriately diluted aliquot of QC in a final volume of 250 µl. Excitation/emission wavelengths were 320/410 nm. The assay reactions were initiated by addition of glutaminyl cyclase. QC activity was determined from a standard curve of b-naphthylamine under assay conditions. One unit is defined as the amount of QC catalyzing the formation of 1 µmol pGlu-bNA from H-Gln-bNA per minute under the described conditions.

In a second fluorometric assay, QC was activity determined using H-Gln-AMC as substrate. Reactions were carried out at 30 °C utilizing the NOVOStar reader for microplates (BMG labtechnologies). The samples consisted of varying concentrations of the fluorogenic substrate, 0.1 U pyroglutamyl aminopeptidase (Qiagen) in 0.05 M Tris/HCl, pH 8.0 containing 5 mM EDTA and an appropriately diluted aliquot of QC in a final volume of 250 µl. Excitation/emission wavelengths were 380/460 nm. The assay reactions were initiated by addition of glutaminyl cyclase. QC activity was determined from a standard curve of 7-amino-4-methylcoumarin under assay conditions. The kinetic data were evaluated using GraFit software.

### Spectrophotometric assay of QC

In this assay, QC activity was analyzed spectrophotometrically using a continuous method, that was derived by adapting a previous discontinuous assay (Bateman, R. C. J. 1989 J Neurosci Methods 30, 23-28) utilizing glutamate dehydrogenase as auxiliary enzyme. Samples consisted of the respective QC substrate, 0.3 mM NADH, 14 mM a-Ketoglutaric acid and 30 U/ml glutamate dehydrogenase in a final volume of 250 µl. Reactions were started by addition of QC and persued by monitoring of the decrease in absorbance at 340 nm for 8-15 min.

The initial velocities were evaluated and the enzymatic activity was determined from a standard curve of ammonia under assay conditions. All samples were measured at 30 °C, using either the SPECTRAFluor Plus or the Sunrise (both from TECAN) reader for microplates. Kinetic data was evaluated using GraFit software.

## Claims

1. A method for diagnosing Alzheimer's Disease (AD), Neurodegeneration in Down's Syndrome (NDS) or Mild Cognitive Impairment (MCI) in a subject, the method comprising:
(a) detecting the amount of glutaminyl cyclase (QC), or an isoform thereof, in a biological sample obtained from said subject; and
(b) comparing the detected amount of QC in the biological sample with an amount of QC characteristic of a normal control;
whereby an elevated amount of QC in said biological sample relative to the normal control is a positive indicator of AD, NDS or MCI.

2. The method of claim 1, comprising:
(a) detecting the amount of glutaminyl cyclase (QC), or an isoform thereof, in a biological sample obtained from said subject; and
(b) further detecting the amount of Aβ N3pE-X,
(c) comparing the detected amount of QC and Aβ N3pE-X in the biological sample with an amount of QC and Aβ N3pE-X characteristic of a normal control;
whereby an elevated amount of QC and Aβ N3pE-X in said biological sample relative to the normal control is a positive indicator of AD, NDS or MCI, and
wherein X is an integer selected from 38, 40 and 42.

3. The method according to any of the preceding claims, wherein said biological sample is serum, plasma, urine or cerebrospinal fluid.

4. The method according to any of the preceding claims, wherein the amount of QC is detected by immunoturbidimetric assay, immunofluorescence, immunodiffusion, enzyme-linked immunosorbent assay (ELISA), radioimmunoassay (RIA), Western blot, protein activity assay, Northern Blot, PCR, high performance liquid chromatography (HPLC), mass spectrometry (MS), gas chromatography (GC), GC-MS, LC-MS, or LC-MS/MS.

5. The method according to any one of the preceding claims, wherein the amount of QC, or an isoform thereof, is detected on the basis of the protein level or the mRNA level of said QC or isoform thereof.

6. The method according to any of the preceding claims, wherein the amount of QC is detected using an antibody that specifically binds to QC, or an isoform thereof.

7. The method according to any of the preceding claims, wherein the amount of QC is detected by measuring the enzymatic activity of QC, or an isoform thereof.

8. The method according to any one of the preceding claims, wherein not only a single form of the N-terminally truncated and pyroglutamated amyloid beta peptides but a combination of Aβ N3pE-42 and/or Aβ N3pE-40 and/or Aβ N3pE-38, and pGluABri and/or pGluADan is detected together with QC.

9. The method according to any one of the preceding claims, comprising:
(a) contacting said biological sample with an antibody that binds to glutaminyl cyclase (QC), or its isoforms ;
(b) allowing the antibody and QC to form an immune complex; and
(c) detecting the amount of immune complex formed as an indication of the amount of QC in said biological sample; and
(d) comparing the detected amount to a sample from normal control subject; whereby a detected amount that is elevated relative to the normal control is a positive indicator of a neurodegenerative disease.

## Patentansprüche

1. Verfahren zur Diagnose der Alzheimer-Krankheit (AD), Neurodegeneration in Down-Syndrom (NDS) oder der Milden Kognitiven Störung (Mild Cognitive Impairment, MCI) in einem Subjekt, wobei das Verfahren umfasst:
(a) das Nachweisen der Menge an Glutaminylcyclase (QC) oder einer Isoform davon in einer biologischen Probe, die von dem Subjekt gewonnen wurde; und
(b) das Vergleichen der nachgewiesenen Menge von QC in der biologischen Probe mit einer Menge der QC, die charakteristisch für eine normale Kontrolle ist;
wobei eine erhöhte Menge an QC in der biologischen Probe relativ zu der normalen Kontrolle ein positiver Indikator für AD, NDS oder MCI ist.

2. Das Verfahren nach Anspruch 1, umfassend:
(a) das Nachweisen der Menge an Glutaminylcyclase (QC) oder einer Isoform davon in einer biologischen Probe, die von dem Subjekt gewonnen wurde; und
(b) weiterhin das Nachweisen der Menge an Aβ N3pE-X,
(c) das Vergleichen der erfassten Menge von QC und Aβ N3pE-X in der biologischen Probe mit einer Menge der QC und Aβ N3pE-X, die charakteristisch für eine normale Kontrolle sind;
wobei eine erhöhte Menge an QC und Aβ N3pE-X in der biologischen Probe relativ zu der normalen Kontrolle ein positiver Indikator für AD, NDS oder MCI ist, und
wobei X eine ganze Zahl, ausgewählt aus 38, 40 und 42, ist.

3. Das Verfahren nach einem der vorhergehenden Ansprüche, wobei die biologische Probe Serum, Plasma, Urin oder Zerebrospinalflüssigkeit ist.

4. Das Verfahren nach einem der vorhergehenden Ansprüche, wobei die Menge der QC mit einem Immunologischen Assay, Immunfluoreszenz, Immundiffusion, Enzymimmunoassay (ELISA), Radioimmunoassay (RIA), Western Blot, Proteinaktivitätstest, Northern Blot , PCR, Hochleistungsflüssigkeitschromatographie (HPLC), Massenspektrometrie (MS), Gaschromatographie (GC), GC-MS, LC-MS oder LC-MS / MS bestimmt wird.

5. Das Verfahren nach einem der vorhergehenden Ansprüche, wobei die Menge der QC oder einer Isoform davon auf der Grundlage des Proteinspiegels oder des mRNA-Spiegels der QC oder einer Isoform davon bestimmt wird.

6. Das Verfahren nach einem der vorhergehenden Ansprüche, wobei die Menge der QC mit einem Antikörper bestimmt wird, der spezifisch an QC oder eine Isoform davon bindet.

7. Das Verfahren nach einem der vorhergehenden Ansprüche, wobei die Menge der QC durch Messen der enzymatischen Aktivität der QC oder einer Isoform davon bestimmt wird.

8. Das Verfahren nach einem der vorhergehenden Ansprüche, wobei nicht nur eine einzige Form der N-terminal verkürzten und Pyroglutamat-tragenden Amyloid-beta-Peptide, sondern eine Kombination von Aβ N3pE-42 und / oder Aβ N3pE-40 und / oder Aβ N3pE-38, und pGluABri und / oder pGluADan zusammen mit QC nachgewiesen werden.

9. Das Verfahren nach einem der vorhergehenden Ansprüche, umfassend:
(a) das Inkontaktbringen der biologischen Probe mit einem Antikörper, der an Glutaminylcyclase (QC), oder deren Isoformen bindet;
(b) das Ermöglichen der Bildung eines Immunkomplexes des Antikörper und der QC; und
(c) das Nachweisen der Menge des Immunkomplexes als Hinweis auf die Menge an QC in der genannten biologischen Probe; und
(d) das Vergleichen der nachgewiesenen Menge mit einer Probe von einem normalen Kontrollsubjekt;
wobei eine nachgewiesene Menge, die im Vergleich zu der normalen Kontrolle erhöht ist, ein positiver Indikator für eine neurodegenerative Erkrankung ist.

## Revendications

1. Procédé de diagnostic de la maladie d'Alzheimer (MA), de la neurodégénérescence dans le syndrome de Down (NDS), ou d'une déficience cognitive légère (MCI) chez un sujet, le procédé comprenant :
(a) la détection de la quantité de glutaminyl cyclase (QC), ou d'une isoforme de celle-ci, dans un échantillon biologique prélevé sur ledit sujet ; et
(b) la comparaison de la quantité détectée de QC dans l'échantillon biologique à une quantité de QC caractéristique d'un témoin normal ;
une quantité élevée de QC dans ledit échantillon biologique relativement au témoin normal étant un indicateur positif de MA, NDS ou MCI.

2. Procédé selon la revendication 1, comprenant :
(a) la détection de la quantité de glutaminyl cyclase (QC), ou d'une isoforme de celle-ci, dans un échantillon biologique prélevé sur ledit sujet ; et
(b) la détection subséquente de la quantité d'Aβ N3pE-X,
(c) la comparaison de la quantité détectée de QC et d'Aβ N3pE-X dans l'échantillon biologique à une quantité de QC et d'Aβ N3pE-X caractéristique d'un témoin normal ;
une quantité élevée de QC et d'Aβ N3pE-X dans ledit échantillon biologique relativement au témoin normal étant un indicateur positif de MA, NDS ou MCI, et
dans lequel X est un nombre entier sélectionné parmi 38, 40, et 42.

3. Procédé selon l'une quelconque des revendications précédentes, dans lequel ledit échantillon biologique est le sérum, le plasma, l'urine ou le liquide céphalorachidien.

4. Procédé selon l'une quelconque des revendications précédentes, dans lequel la quantité de QC est détectée par un dosage immunoturbidimétrique, par immunofluorescence, par immunodiffusion, par ELISA (enzyme-linked immunosorbent assay), par un dosage radioimmunologique (RIA), par un transfert de type Western, par un dosage de l'activité protéique, par transfert de type Northern, par PCR, par chromatographie en phase liquide haute performance (CLHP), par spectrométrie de masse (SM), par chromatographie en phase gazeuse (CG), par CG-SM, par CL-SM, ou par CL-SM/SM.

5. Procédé selon l'une quelconque des revendications précédentes, dans lequel la quantité de QC, ou d'une isoforme de celle-ci, est détectée sur la base du niveau de protéines ou du niveau d'ARNm de ladite QC ou d'une isoforme de celle-ci.

6. Procédé selon l'une quelconque des revendications précédentes, dans lequel la quantité de QC est détectée en utilisant un anticorps qui se lie spécifiquement à la QC, ou à une isoforme de celle-ci.

7. Procédé selon l'une quelconque des revendications précédentes, dans lequel la quantité de QC est détectée par mesure de l'activité enzymatique de la QC ou d'une isoforme de celle-ci.

8. Procédé selon l'une quelconque des revendications précédentes, dans lequel non seulement une forme simple des peptides bêta amyloïdes pyroglutamatés et à troncature N-terminale, mais aussi une combinaison d'Aβ N3pE-42 et/ou Aβ N3pE-40 et/ou Aβ N3pE-38, et pGluABri et/ou pGluADan sont détectées conjointement à la QC.

9. Procédé selon l'une quelconque des revendications précédentes, comprenant :
(a) la mise en contact dudit échantillon biologique avec un anticorps qui se lie à la glutaminyl cyclase (QC), ou ses isoformes ;
(b) le fait de laisser l'anticorps et la QC former un complexe immun ; et
(c) la détection de la quantité du complexe immun formé en tant qu'indication de la quantité de QC dans ledit échantillon biologique ; et
(d) la comparaison de la quantité détectée à un échantillon d'un sujet témoin normal ;
une quantité détectée élevée relativement au témoin normal étant un indicateur positif d'une maladie neurodégénérative.
